Europäisches Patentamt

**European Patent Office**

Office européen des brevets

(19)

(11) Publication number: **0 075 860**
**A2**

(12) # EUROPEAN PATENT APPLICATION

(21) Application number: **82108784.8**

(22) Date of filing: **22.09.82**

(51) Int. Cl.³: **A 61 B 17/36**

(30) Priority: **24.09.81 US 305175**
**24.08.82 US 409609**

(43) Date of publication of application:
**06.04.83 Bulletin 83/14**

(84) Designated Contracting States:
**AT BE CH DE FR IT LI LU NL SE**

(71) Applicant: **Morris, James Robert**
**8914 Wald Road**
**Houston Texas 77034(US)**

(72) Inventor: **Morris, James Robert**
**8914 Wald Road**
**Houston Texas 77034(US)**

(74) Representative: **Patentanwälte Grünecker, Dr.**
**Kinkeldey, Dr. Stockmair, Dr. Schumann, Jakob, Dr.**
**Bezold, Meister, Hilgers, Dr. Meyer-Plath**
**Maximilianstrasse 43**
**D-8000 München 22(DE)**

(54) **Microsurgical laser.**

(57) A self-contained microsurgical laser which has a housing adapted for mounting on a surgical microscope. Mounted within the housing are an infrared laser source and a visible laser source. Means are provided in the beam path of the infrared laser for interrupting the beam and for polarizing and attenuating the beam. Beam expanding means are provided in the beam paths. Wave front correction means are provided in the path of the visible laser beam so that its focal point is coincident with the focal point of the infrared laser beam. Means are provided for superimposing the beam of the visible laser onto the beam of the infrared laser. Power level detecting means are provided. Means are provided for deflecting the combined laser beams in the X and Y directions in a plane perpendicular to the combined beams. Means are provided for focusing the combined beams onto a spot of less than 200 microns in diameter at the focal plane of the focusing means. A method for repairing torn or severed body structure comprising exposing the surfaces to be joined to an infrared laser source for a period of time sufficient to effect the repair.

./...

FIG. 2

## METHOD FOR REPAIRING BODY STRUCTURES
## AND A MICROSURGICAL LASER USEFUL THEREFORE

### BACKGROUND OF THE INVENTION

The present invention is directed to a microsurgical laser useful in the performance of surgical techniques such as the cutting and ablating of flesh and the anastomosis of vessels. The invention is also directed to a method for repairing severed or torn body structures.

The use of lasers in surgery has been known since the 1960s. One of the first such uses was a procedure for the reattachment of retinas. The use of lasers in the anastomosis of vessels was first contemplated in the middle 1960s. The development of such a technique is highly desirable as the microsuture technique of small vessel repair is time consuming and requires vascular occlusion for periods which are longer than the safe limit in the case of organ such as the brain. In addition, the suture technique is difficult and often impossible with vessels smaller than 0.5 mm.

Until 1979, the only practical use of lasers in surgical techniques involving vessels was utilization of the hemostatic effect of the laser beam. Experiments on the repair of small blood vessels (0.3 to 1.0 mm) in rats using a Neodymium-YAG laser was reported in Surgery, volume 85, page 684 (1979) and in the Journal of Micro-Surgery, page 434 (May/June 1980).

These techniques of anastomosis and other microsurgical techniques involving lasers were hampered by the large size of the lasers and their inability to focus on a spot size small enough to allow effective repair of small vessels.

Conventional lasers currently in use in operating rooms are large bulky instruments requiring supporting vacuum pump, cooling system, and gas cylinders. Such units weigh in excess of 200 lbs and thus are not easily moved. The laser itself is mounted in a unit sitting on the floor and the beam is transmitted through optical fibers or an articulated arm to an optical apparatus which allows manipulation of the beam and focusing. This apparatus is mounted on a surgical microscope through which the surgeon views the work area. The beam is manipulated by the surgeon through the use of a control stick and the surgeon must constantly use one hand to control and position the laser beam.

Such lasers in addition to their bulk and requirements for support equipment are unable to produce a spot size smaller than 500 microns at their focal plane. The tissue destruction zone is dependent on the spot size and the larger the spot size the more extensive and widespread is the tissue destruction occurring. The spot size is especially important in the anastomosis of small vessels less than one millimeter in diameter.

The microsurgical laser of the present invention avoids these drawbacks. The microsurgical laser of the instant invention is small, approximately 6" x 6" x 15", lightweight, less than 12 lbs, and self-contained. There is no requirement of vacuum pumps, cooling systems, or compressed gas cylinders. The microsurgical laser is adapted to be mounted directly on a surgical microscope.

Through the use of microprocessors the movement of the beam required by the surgeon can be programmed prior to carrying out the actual surgical technique and then repeated thus freeing the hands of the surgeon.

The spot size of the beam produced by the laser of the present invention is smaller than 200 microns. This results in less tissue being destroyed as the beam is used and allows the device to be used to repair smaller vessels than heretofore possible. Another advantage of the small spot size is that the same power density may be obtained using a lower power laser.

## SUMMARY OF THE INVENTION

The present invention is a self-contained microsurgical laser. The laser comprises a housing adapted to be mounted on standard surgical microscope, an infrared laser source and a visible laser source. Means for interrupting the infrared laser beam and for polarizing and attenuating the infrared laser beam are provided in the beam path. Beam expanding means are provided in the beam paths. Wavefront correction means are placed in the visible laser beam path so that its wavefront is deformed such that its focal point will be coincident with that of the infrared laser beam after passing through the focusing lens. A beam splitter is provided in the beam path of both lasers which serves as a means to superimpose the path of the visible laser beam onto the beam path of the infrared laser beam. Means for measuring the power level of the infrared laser beam are provided. Means for deflecting the combined laser beams in the X and Y directions in a plane perpendicular to the combined beams as they emerge from the focusing lens are also provided. A final lens is provided to focus the combined laser beams onto a spot of less than 200 microns in diameter at the focal plane of the lens.

The microsurgical laser is particularly well suited for effecting the repair of torn or severed body structures. The method of repair comprises generating an infrared laser beam, positioning the body structure to be repaired at the focal plane of the laser beam. The two surfaces to be joined are brought into close proximity to one another. The laser beam is focused onto a spot less than 200 microns in diameter and directed onto the juncture of the two surfaces for a time sufficient to effect the repair.

### BRIEF DESCRIPTION OF THE DRAWINGS

FIGURE 1 is a schematic showing the beam paths of the infrared and visible laser beams and the various components.

FIGURE 2 is a top view of the surgical laser.

FIGURE 3 is a side view of the surgical laser.

FIGURE 4 is a block diagram of the control circuitry.

## DETAILED DESCRIPTION

### Laser Sources

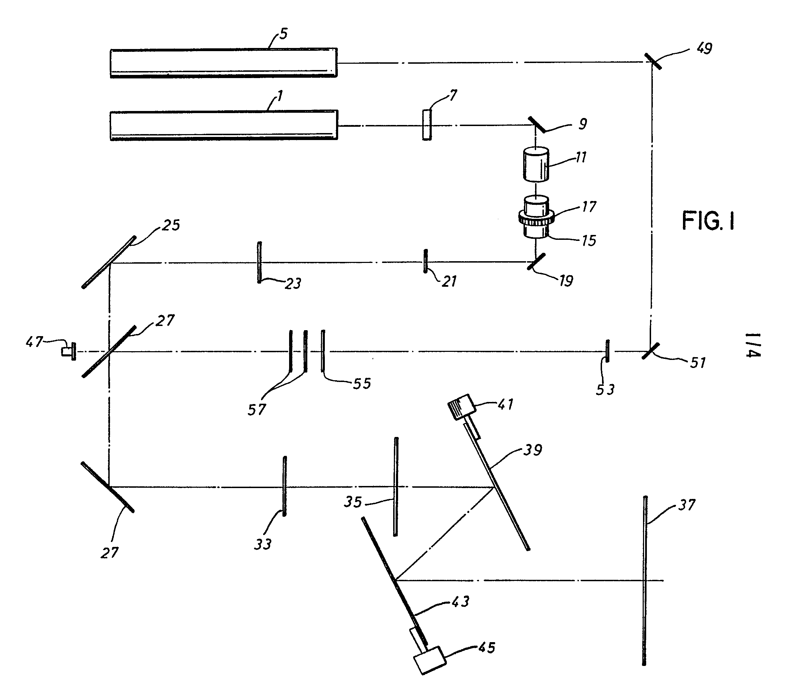

As shown in Figure 1, a means for generating an infrared laser beam 1 is mounted inside a lightweight essentially rectangular housing (not shown) approximately 6" x 6" x 15". The preferred source of the infrared laser beam is $CO_2$ waveguide laser emitting a beam having a wavelength of 10.6 microns. The preferred power range of the $CO_2$ laser is from about 5 to about 10 watts. The housing may be of any lightweight material of sufficient strength to support the internal components of the laser. For example, an aluminum manufactured structural honeycomb sandwiched between parallel plates may be used to get the desired characteristics of extremely low weight and sufficient strength.

A means for generating a visible laser beam is also mounted in the housing parallel to the infrared laser source. The preferred source for this beam is a helium-neon (HeNe) laser emitting a beam having a wavelength of 6328 A. HeNe lasers with outputs in the range of 2-5 milliwatts are preferred.

### Infrared Beam Path

The beam emitted from the infrared laser 1 passes through a beam interrupter 7 which is electronically controlled. This interrupter allows the surgeon to position the laser and practice and/or program the movements required with the infrared beam off, using only the harmless visible beam. The preferred beam interrupter is a simple shutter mechanism, however, other means may be employed.

After passing through the interrupter, the infrared beam strikes a turning mirror 9 which deflects the beam 90°. The number and position of such turning mirrors are depended upon the geometry and configuration of the components of the laser. The position of the mirrors and other elements as shown in the accompanying figure will be described in this application with no intent to limit the instant invention to that particular configuration. The preferred mirror material is pyrex glass coated with aluminum silicate with an overcoat of a hard dielectric material. Other mirrors such as silver or gold coated plated pyrex may also be used.

The beam next passes through a polarizer 11. The preferred means for polarizing the beam is a Brewster plate. The infrared beam next passes through a second polarizer 15. Again, the preferred polarizer is a Brewster plate. By rotating this polarizer, the polarized beam from the first polarizer is attenuated and the power level of the infrared beam may thus be controlled. A gear 17 driven by servo motor turns the second polarizer in response to the controls operated by the surgeon.

The infrared beam after passing through the second polarizer and striking turning mirror 19 passes through a beam expander 21 causing the wave path of the beam to diverge. The beam expander is a negative lens approximately 1/4" in diameter and can be made of zinc selenium (ZnSe), barium fluoride (BaFl) or germanium (Ge). Lens as used herein is to be interpreted in its broad meaning and may indicate a single optical component or a combination of such components aggregated to achieve the desired results.

The infrared beam next passes through a columnator lens 23 which can also be made of ZnSe, BaFl or Ge and is approximately 1/2" in diameter. After passing through this lens the beam is essentially non divergent. The beam leaving lens 23 is approximately 1/2" in diameter.

After striking another turning mirror 25 the infrared beam passes through a beam splitter 27 set at a 45° angle to the path of the infrared beam. The beam splitter is made from germanium. The beam splitter is anti-reflection coated at 45°. Ninety-five percent of the infrared beam passes through the beam splitter. The remaining 5% is reflected to a photo-cell 47 which measures the power level of the infrared beam. The measured power level is displayed on the controls of the instrument.

Visible Beam Path

The beam emitted from the visible wavelength laser 5 strikes two turning mirrors 49 and 51, each set at a 45° angle to the path of the beam. The beam next passes through a beam expander consisting of a negative lens of quartz or fused silica causing the beam to diverge. The beam next passes through a columnator lens 55.

Following the columnator lens, the beam passes through a wave front correction means 57 which deforms the wave front of the visible beam so that upon passing through the final focusing lens, its focal point will be coincident with that of the infrared beam, thus allowing the surgeon to accurately position the infrared beam by positioning the visible beam. The wave front correction means illustrated in Figure 1 is composed of two positive lenses of fused silica or quartz.

The visible beam next strikes the beam splitter 27 which is set at a 45° angle to the path of the visible beam. Ninety-five percent of the visible laser beam is reflected by the beam splitter. The portion thus reflected is superimposed on the path of the infrared beam.

## Combined Beam Paths

The combined beams after leaving the beam splitter strikes turning mirror 29 and then pass through a beam expander 33 consisting of a negative ZnSe lens approximately 3/4" in diameter. The resulting divergent combined beams next pass through a columnator 35 which is a ZnSe lens approximately 1-1/2" in diameter rendering the beams parallel again and approximately 1-1/2" in diamter. Thus the beam is expanded from its original diameter of .06". This expansion in the beam diameter allows for the final focusing lens to be significantly faster than those known in the prior art. For example, the f number of the focusing lens of the prior art devices was in the range of 10 to 12. Using the optical components and arrangement of the present invention gives f numbers in the range of 3 to 5.

As shown in Figure 1, the combined beams next strike mechanisms for manipulation of the beam in the X and Y directions. The preferred mechanisms are galvanometer scanning mirrors consisting of mirrors 39 and 43, made from berillium coated with aluminum silicate and a hard dielectric, pivoted on shafts extending from galvanometers 41 and 45. Berillium is the preferred material due to its light weight allowing the mirrors to be postioned by the galvanometers. One such mirror allows the beam to be translated in the X direction in a plane perpendicular to the axis of the combined beams after the beam leaves the final focusing lens. The second mirror allows similar movement in the Y direction.

The movement of the galvanometer scanning mirrors, and thus control of the positioning of the laser beam, is controlled by the surgeon through the use of a control stick which is connected to appropriate circuitry to provide the requisite output voltage to the galvanometers to produce the required movement. Feedback circuits can be provided to feed the voltage parameters into memory circuits which allow the surgeon to program a particular pattern of motion while using only the visible beam and to then through the use of the memory circuits and the galvanometer scanning mirrors repeat that exact pattern while using the infrared beam. This feature frees the hands of the surgeon for other task during the time the infrared beam is in use. In addition movement of the scanning microns can be controlled by a foot operated track ball.

After leaving the second galvanometer scanning mirror, the combined beams pass through a final focusing lens 37. The preferred lens is a ZnSe lens with a 6 to 8 inch focal length. The lens is capable of producing a spot size of less than 200 microns at its focal plane. Spot sizes as small as 75 microns are achievable with the instant invention. Preferably the lens will be interchangeable to match the focal length of the microscope lens. The focusing lens may be mounted on a motorized translation stage to aid in final focus control.

## DESCRIPTION OF A PREFERRED EMBODIMENT

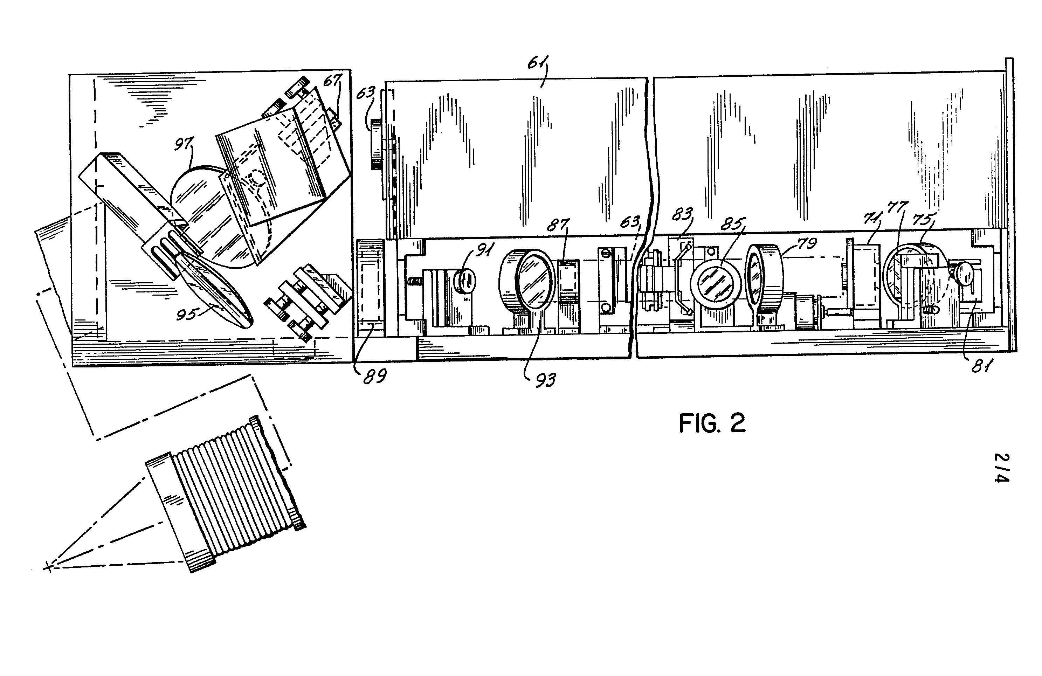

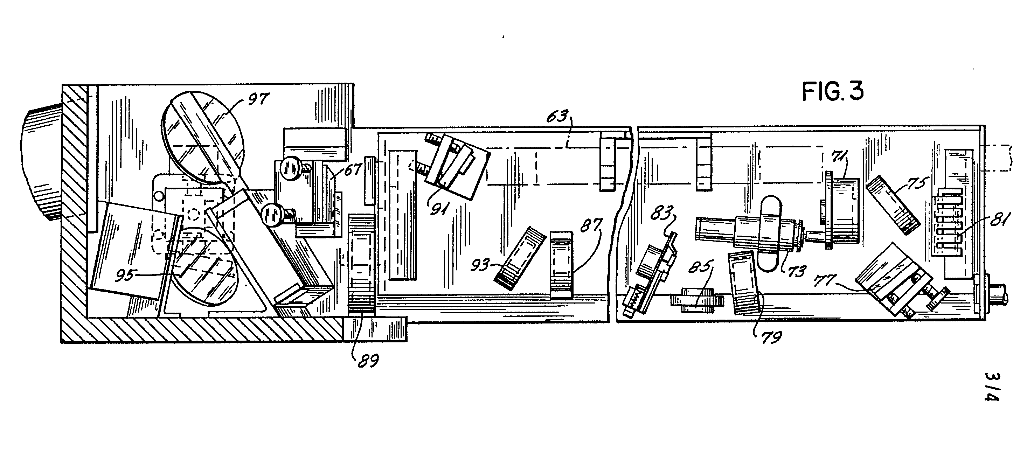

The actual physical arrangement of the various component may be seen in Figures 2 and 3.

Referring to Figures 2 and 3 the $CO_2$ laser 61 and HeNe laser 63 may be seen. The infrared beam 65 strikes two turning mirrors 67 and 69. The infrared beam next encounters polarizer 71 operated by servo motor 73. The preferred polarizer is a wire grid polarizer such as manufactured by PTR Optic. The infrared beam is again turned by means of two mirrors 75 and 77. The beam next strikes the radiometer window or beam splitter 79 which reflect 5% of the infrared beam to the radiometer 81 which measures the power level of the infrared beam. The remaining 95% of the infrared beam next encounters a shutter mechanism 83 which, when closed, allows focusing and alignment of the surgical laser using only the harmless visible beam. When the shutter is closed the infrared beam is reflected into beam dump 85 where it is absorbed. The infrared beam next passes through a 10X beam expander made up of a negative lens 87 and a positive lens 89.

The visible laser beam strikes turning mirror 91 which directs the beam to beam splitter 93 which is located in the infrared beam path between the two lenses of the expander. This beam splitter is coated with Geranium so that it allows approximately 95% of the infrared beam to pass through it and reflects 100% of the visible beam. The reflected visible beam is thus superimposed on the infrared beam.

The combined beams next strike galvonometer mirrors 95 and 97 which are controlled to manipulate the X and Y coordinates of the combined beams. Finally the combined beams pass through a final focusing lens 99.

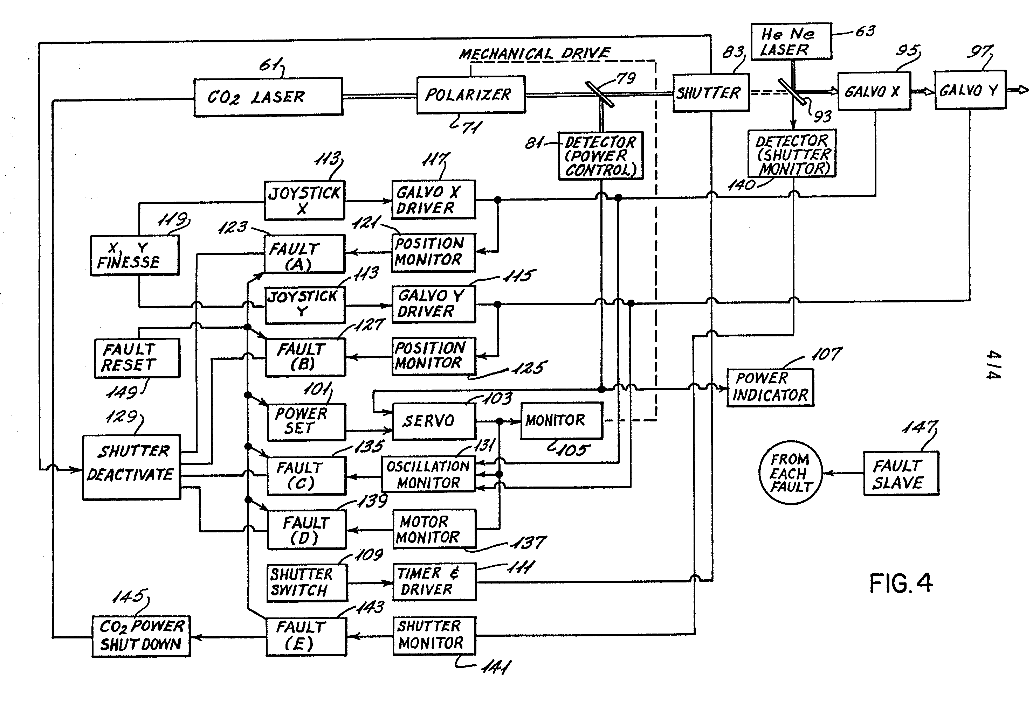

Figure 4 is a block diagram showing control circuitry for the surgical laser exemplified in Figures 2 and 3. The desired $CO_2$ laser power is selected by means of the power set which uses a potentiometer to produce a voltage which represent a desired laser power output level. The servo circuit 103 compares the output of radiometer 81 with this output and adjusts the polarizer 71 via the motor 105 until the servo condition is satisfied. The actual power is indicated on the control module by the power indicator 107.

The shutter 83 is operated by the shutter switch 109 which is a single pole, double throw switch which activates (opens) the shutter when depressed. Timer and driver circuit 111 allows the selection of a particular exposure or pulse length causing the driver to activate the shutter for the desired time.

The positioning of the combined laser beams is controlled by means of a joystick. The joystick mechanically operates a potentiometer 113. The signal from this poteniometer is translated into physical motion of the galvonometer mirrors by X and Y galvonometer drivers 115 and 117.

The X-Y finesse circuit 119 controls the degree of movement of the combined beams at the focal plane per unit movement of the joystock. Thus, when delicate work is being performed this circuit allows small movement of the beam when the joystick is moved through a large angle giving the surgeon greater control. In addition this circuit is used to prevent movement of the beam outside the surgeon's field of view when high magnification is being used.

In addition to the circuits just described which control the operation of the surgical laser, a number of circuits are provided to insure the safe operation of the surgical laser to prevent injury to the patient in the event of a malfunction of one of the control circuits.

With the exception of a malfunction in the shutter all fault circuits operate to close the shutter if a fault is detected. Position monitor circuits 121 and 125 monitor excessive angular motion of the X and Y galvonometer mirrors. Such excessive angular motion may occur during power up. If excessive angular motion of either of the two galvonometer mirrors is detected, fault circuits 123 and 127 operate to close the shutter through the shutter deactivate circuit 129. It is also possible that oscillations may begin to occur in one or both of the galvonometer mirrors or in the servo motor circuit 103. Such oscillations are detected by oscillation monitor circuit 131 and fault circuit 135 operates to close the shutter through the shutter deactivate circuit 129. The final fault system acting through the shutter deactivate circuit is the motor monitor 137 and fault circuit 139. This circuit detects and enunciates a fault if the motor fails to respond to the servo circuit.

Failure of the shutter control is detected by the shutter monitor 141 from a signal from radiometer 140. If the shutter fails to close properly the radiometer detects the portion of the infrared beam reflected by the beam splitter 93. The shutter timing pulse length is compared with actual opening time of the shutter by the shutter monitor circuit 141 and if there is a discrepancy the fault is enunciated through fault circuit 143 and the power to the $CO_2$ laser is shut off through $CO_2$ power shut down circuit 145.

All the fault circuits act through the fault slave circuit 147 to enunciate by LED indicator that a fault has occurred. Circuit 149 acts to reset all fault lights.

Use of the Microsurgical Laser

In use, the surgeon first locates the laser beam in the desired position using the visible beam only with the infrared beam blocked by means of the beam interrupter. The beam is manipulated by the surgeon using a control stick which operates the galvanometer scanning mirrors.

If desired, the surgeon may manipulate the beam in a desired pattern using only the visible beam. Feedback circuits supply the voltage output being applied to the galvanometer scanning mirrors to a memory circuit. The surgeon can then activate the infrared beam and the exact motion of his previous pattern is executed leaving the surgeon's hands free for use in the operative field. This feature decreases the need for assistants in the operating room.

Due to the small spot size produced by the instant invention, it is especially useful in the anastomosis of vessels less than 1 mm in diameter. The exact nature of the mechanism of the bonding brought about by exposure to the laser beam is not known. The same technique is useful in the repair of body structures other than blood vessels. Among such structures which may be repaired are arteries, veins, vas deferns, fallopian tubes, intestines, bowels, nerves, muscles and heart valves. The technique will be described more particularly with respect to the repair of small vessels that have been severed.

The technique is carried out by bringing the two ends of the severed vessel into close proximity by means of clamps. Two or three sutures are placed in the vessel to hold the ends in proper alignment. Generally the satures are placed 120° apart around the circumference of the artery.

The vessel is positioned at the focal plane of the laser. The laser beam is then positioned using the visible beam only. The infrared beam is then turned on for a predetermined length of time. The length of exposure is generally from about .2 to .6 seconds with an infrared laser output of 50-100 milliwatts. The beam may be stationary or may sweep across the joint. The vessel is then rotated and the procedure repeated. The number of exposures to the laser beam is depended upon the size of the vessel to be joined. Typically two to four exposure are needed to complete the repair.

After the repair is completed the sutures may be removed. Using this technique, the amount of time needed to affect the repair is reduced from the 30 minutes to one hour required for standard microsurgical techniques to two to three minutes.

Repair techniques utilizing the instant invention with its small spot size avoid the problem of the vessel being constricted in the area of the repair. This constriction is the result of thermal dehydration of tissue near the repair. The amount of such dehydrated tissue is reduced by the decrease in spot size.

Test on severed femoral arteries of live rats have proven the technique to be successful in repair of arteries between .7 and 1.5 mm in diameter. A comparison of the patency rates have shown the technique to give superior results to those obtained using conventional microsurgical techniques.

The repair technique has also been tested successfully in the repair of vas deferens and fallopian tubes of rats. It has also successfully been used to repair intestines and bowels. The technique is equally applicable to the repair of vessels which are not severed but which have been torn or punctured. The surfaces to be joined are brought in to close proximity in the same manner as described above in the case of a totally severed vessel. The surfaces are then exposed to the infrared laser beam for a time sufficient to effect the repair.

In addition to the repair of vessels and other tube structures, the technique is applicable to the repair of other torn or cut body structures such as heart valves, nerves, and muscles.

## ALTERNATE EMBODIMENT

It is possible to have the two laser beam sources exterior to the housing mounted on the surgical microscope. In such a situation the laser sources could be of the conventional type now used in operating rooms. The beams would be transmitted to the optical component package by an articulated arm or by optical fibers. Mounted on the optical microscope would be a housing containing polarizing and attenuating means such as those described previously. Beam expansion is also carried out in the same manner as described above. Positioning of the beam is controlled by galvanometer scanning mirrors. A final focusing element is provided which is capable of producing a spot size of less than 200 microns at the focal plane. Such a device, although it does not provide the compactness of the preferred embodiment does due to its small spot size allow its use for the repair procedures described below.

Modifications and variations of the invention will be apparent to those skilled in the art. It is the applicant's intention in the following claims to cover all such equivalent modifications and variations as fall within the true spirit and scope of the invention.

WHAT IS CLAIMED:

1. An instrument suitable for focusing a laser beam to a small spot in a focal plane characterized in that said instrument comprises:

a laser source for producing a laser beam;

means associated with the laser beam source for expanding the cross-section of the laser beam and for collimating the expanded laser beam;

a lens for focusing the expanded laser beam to a small spot on said focal plane, said focusing lens having a cross-section larger than the cross-section of the expanded laser beam;

means located between the expanded and collimating means and the focusing lens for moving the expanded beam laterally in relation to the focusing lens to allow the small spot to be adjusted and moved in the focal plane.

2. An instrument according to claim 1 further characterized in that said beam moving means comprise two scanning galvanometer mirrors.

3. An instrument according to claims 1 and 2 further characterized in that a means for interrupting the laser beam is located in the path of said laser beam.

4. An instrument according to claim 3 further characterized in that power level detection means are associated with said laser beam, said power level detection means being located so that the power level of said laser beam can be monitored with said laser beam blocked by said interrupting means.

5. An instrument according to claims 1 through 4 further characterized in that means for attenuating the laser beam to control its power are provided.

6. An instrument according to claims 4 through 5 further characterized in that said power level detection means comprises:

a radiometer window located at the path of said laser beam capable of trasmitting the major portion of said laser beam and of reflecting a minor portion of said laser beam;

a radiometer mounted so as to intercept the reflected laser beam; and

circuitry to translate the output from said radiometer into an indication of power level.

7. An instrument according to claims 1 through 6 further characterized in that said laser beam source is a $CO_2$ laser producing an infrared laser beam.

8. An instrument according to claim 7 further characterized in that a visible laser beam source producing a visible laser beam is provided and means for aligning said visible beam on said infrared laser beam are also provided so that said infrared laser beam can be located in said focal plane using the visible laser beam.

9. A method for the repair of body structures characterized in that it comprises:

generating an infrared laser beam;

positioning the body structure to be repaired at the focal plane of said laser beam;

bringing the surfaces to be joined into close proximity with one another;

focusing said infrared laser beam to a spot less than 200 microns in diameter; and

directing said beam spot on to the juncture of the surface to be repaired for a time sufficient to effect the repair.

10. The method of claim 8 further characterized in that said infrared laser beam is located at the juncture of the surfaces to be repaired by directing a visible laser beam, which is co-linear with and which has a focal point coincident with said infrared laser beam, at the juncture of the surfaces to be repaired.

11. A method according to claims 9 through 10 further characterized in that said body structures are blood vessels less than 1 milimeter in diameter.

FIG. I

1/4

0075860

FIG. 2

FIG.3

FIG. 4

MECHANICAL DRIVE

CO₂ LASER — 61

POLARIZER — 71

79

SHUTTER — 83

He Ne LASER — 63

93

GALVO X — 95

GALVO Y — 97

DETECTOR (POWER CONTROL) — 81

DETECTOR (SHUTTER MONITOR) — 140

JOYSTICK X — 113

GALVO X DRIVER — 117

X, Y FINESSE — 119

FAULT (A) — 123

POSITION MONITOR — 121

JOYSTICK Y — 113

GALVO Y DRIVER — 115

FAULT RESET — 149

FAULT (B) — 127

POSITION MONITOR — 125

POWER SET — 101

SERVO — 103

MONITOR — 105

POWER INDICATOR — 107

SHUTTER DEACTIVATE — 129

FAULT (C) — 135

OSCILLATION MONITOR — 131

FROM EACH FAULT

FAULT SLAVE — 147

FAULT (D) — 139

MOTOR MONITOR — 137

SHUTTER SWITCH — 109

TIMER & DRIVER — 111

CO₂ POWER SHUTDOWN — 145

FAULT (E) — 143

SHUTTER MONITOR — 141

4/4

0075860